# EUROPEAN PATENT APPLICATION

(11) **EP 4 098 326 A1**
(43) Date of publication of application: **07.12.2022**
(21) Application number: 21177357.7
(22) Date of filing: 02.06.2021
(51) Int. Cl.: A61Q 5/02, A61Q 5/12, A61Q 19/10, A61K 8/46, A61K 8/60, A61K 8/42, A61K 8/365, A61K 8/02, A61K 8/19

(54) **UNIT DOSE FOR COSMETIC COMPOSITION COMPRISING AN EFFERVESCENT SYSTEM**

(71) Applicant: Dalli-Werke GmbH & Co. KG, 52224 Stolberg (DE)
(72) Inventor: WIERCZIMOK, Dirk, 52076 Aachen (DE); HELMIN, Marta, 52062 Aachen (DE); RÜBENER, Thomas, 52531 Übach-Palenberg (DE); NICKEL, Janine, 67697 Otterberg (DE); SCHARRER, Andreas, 68623 Lampertheim (DE); REHFELDT, Iris, 67316 Carslberg (DE); THIEL, Rolf, 66123 Saarbrücken (DE)
(74) Representative: f & e patent

(57) **Abstract**

The present invention refers to tablets or unit doses of cosmetic or body / hair care compositions suitable for preparing a liquid ready-to-use cosmetic or body / hair care composition, said liquid compositions as well as the use of said tablet or unit doses for preparing said liquid compositions.

## Description

The present invention relates to tablets or unit doses comprising an effervescent system suitable for preparing a liquid or jelly cosmetic composition, in particular a body care or hair care composition, to a liquid or jelly composition prepared by dissolving said tablet or unit dose in water and to the use of such tablets or unit doses for preparing said liquid or jelly compositions.

For laundry or dishwashing tableted detergent compositions or detergent compositions in unit portions have become increasingly popular as they are easy to handle and avoid over- or under-dosing and spillage of detergent. For cosmetic, body or hair care applications, like e.g. shampoo, shower or bath compositions, hand or body wash compositions liquid or jelly cleaning compositions are often more suitable. Most of these cleaners are offered as aqueous liquid cleaning compositions comprising water as the main ingredient. Using said compositions thus involves carrying and transporting significant amounts of water as well as using a huge number of mostly "one-way" plastic containers. Therefore, in the present it is discussed to provide detergent compositions in form of tablets or unit doses provided in suitable portions for preparing such cleaning compositions "ready-for-use" by the consumers themselves. This can be done by adding said tablet or unit dose to a predetermined amount of water.

Detergent compositions in the form of tablets or solid unit doses offer advantages in storage, transport and packaging. However, one major disadvantage is that tablets and other unit portions are usually compressed or compacted and therefore do not always disintegrate, dispense or dissolve satisfactorily in water. This might result in an inefficient delivery of the active agents or to any remainder of the tablet in the water. One solution for this problem presented in the prior art is the incorporation of effervescent systems into a tablet, which form gas upon contact with water and thereby contribute to improved disintegration, dissolution and dispersion of the tableted or portioned composition.

The use of effervescent systems advantageously allows the very rapid disintegration, dispensing and dissolution of the solid dosage forms, such as tablets and other unit portions. In addition, after dissolving, minimal to none manipulation is needed in order to ensure appropriate mixing of the constituents including the active agents of the solid dosage compositions, in particular tablets, such that uniform applications can be assured with the reconstituted aqueous solutions, including water softening, cleaning and detergent solutions.

However, tablets/unit portions of detergent compositions comprising effervescent systems based on an acid-base system are sometimes difficult to store, especially in a humid or moist environment as too high air humidity can already start the effervescent reaction which results in physical instability of the tablet or unit portion.

Thus, cosmetic, body or hair care compositions comprising effervescent systems which are intended to be offered as tablets/unit portions have to be carefully balanced in view of storage stability, dissolution properties and performance and further to aesthetic and acceptance aspects.

WO-A-03/062360 describes a detergent tablet including an effervescent system, polyethylene glycol and an organic compound with two polar groups, such as 1,6-hexanediol for improving the dissolution of the components of the formulation, for example.

WO-A-2012/045907 describes compositions comprising between 20 percent and 30 percent by weight of sodium lauryl sulfate and an effervescent disintegrant adjuvant formed by citric acid and sodium carbonate or sodium bicarbonate.

WO-A-2016/087639 describes effervescent detergent compositions comprising pectin as storage and disintegration supporting material.

US-A-20180105766 describes detergent tablets including an effervescent system based on an organic acid and an alkaline carbonate or bicarbonate, an alkylsulfate-type anionic surfactant and a disintegrating agent.

EP 2 285 7001 A1 describes a bathing tablet comprising an organic acid, polyethylene glycol, an anhydride and a bicarbonate and a method for its preparation.

WO-A-16170338 refers to a solid hair conditioner composition comprising a conditioner concentrate and an effervescent system.

WO-A-18000068 discloses a hair care formulation concentrate comprising a cellulose based cationic polymer and four different polyalkylene glycols.

In WO-A-19002374 a solid cleaning cosmetic composition is described comprising at least 70wt.% microcrystalline cellulose and at least two particular surfactants.

WO-A-19147496 refers to a personal care cleaning product in tablet form comprising surfactants, starch, alcohol and water.

WO-A-20114679 discloses a solid cosmetic cleansing composition comprising an anionic surfactant, an effervescent system and a further disintegration system chosen from a polysaccharide.

WO-A-20210184 describes a cleansing personal care formulation comprising sodium bicarbonate, an organic acid and a surfactant in given weight ratios, wherein bicarbonate is clearly in excess.

The object of the present invention is to provide a cosmetic, a body care or hair care composition in dry, pre-portioned, compact, weight and space saving form allowing to prepare a liquid or jelly composition by the consumers themselves in an easy and plastic waste avoiding manner. The liquid or jelly composition should be preparable fast, non-elaborately and without any solid, greasy or unattractive remainders in the liquid composition. The resulting composition can be used for cosmetic, body care or hair care application.

This object is met by the cosmetic or body / hair care compositions in form of a tablet or unit dose for preparing a liquid composition according to the invention. The tablet or unit dose for preparing the body / hair care or cosmetic compositions according to the present invention comprise an effervescent system, a nonionic surfactant and a further chelating agent, which is different from citric acid /citrate, preferably an amino acid-based chelating agent.

According to the present invention it has been found that a body / hair care or cosmetic composition in form of a tablet or a unit dose comprising an effervescent system can be suitably stored and readily dissolved in water resulting in a ready-to-use liquid body / hair care composition, e.g. for cleaning the skin of face, hands, feet or the whole body, or hair, if said tablet / unit dose comprises besides said effervescent system (i) at least one anionic surfactant (ii), preferably selected from C₆₋₂₀ alkyl sulfosuccinate, C₆₋₂₀ alkyl sulfoacetate or C₆₋₂₀ alkyl isethionate, at least one nonionic surfactant (iii), preferably selected from any C₆₋₂₀ alkyl(poly)glucoside.

The terms "comprising", "comprises" and "comprised of" as used herein are synonymous with "including", "includes" or "containing", "contains", and are inclusive and open-ended and do not exclude additional, non-recited members, elements or method steps. It will be appreciated that the terms "comprising", "comprises" and "comprised of" as used herein comprise the terms "consisting of", "consists" and "consists of".

### Effervescent system

The term "effervescent system" relates to a combination of compounds or mixture of compounds which result in the generation and release of a gas when administered to a liquid or when in contact with a liquid. Any effervescent system known in the art may be used according to the invention. Preferably, the term "effervescent system" refers to a system comprising at least one acidic and at least one basic (= alkaline) component, preferably components capable of reacting to form a gaseous species, such as for example carbon dioxide or oxygen, upon contact with a liquid, e.g. water. Examples of suitable systems are disclosed in DE 2 132862, EP 0 286 085 A1, US 2008/0274931 A1, WO 96/33694 and US 2004/0116317 A1.

In the tablet or unit dose composition according to the invention the effervescent system is preferably present in an amount ranging from 25 to 95 wt.-%, dependent from the type of liquid composition to be prepared, e.g. a shampoo, hair conditioner, shower gel, bathing composition, facial cleanser, hand or body cleansing or any other suitable cosmetic composition. Such a tablet or unit dose body / hair care or cosmetic composition may comprise the effervescent system in an amount of 30 to 90 wt.-%, preferably 35 to 87,5 wt.-%, more preferred 40 to 85 wt.-%, even more preferred 45 to 82,5 wt.-% or 50 to 80 wt.-% of the solid body / hair care or cosmetic composition Each individual ingredient of the effervescent system according to the invention is soluble in water of 20°C up to at least 5 gram per 100 ml.

The acidic component is preferably selected from tricarboxylic acids, dicarboxylic acids, including unsaturated dicarboxylic acids, inorganic acids, and salts thereof or derivatives thereof, e.g. naturally occurring unsaturated variants of the acids cited below (like e.g. aconitic acid, an unsaturated variant of citric acid). Examples of suitable acidic components of the present invention include citric acid, isocitric acid, oxalic acid, tartaric acid, malonic acid, succinic acid, glutaric acid, malic acid, adipic acid, amidosulfonic acid, maleic acid, glutaconic acid, mono sodium citrate, di sodium citrate; one particularly preferred acidic component is citric acid or salts thereof, even more preferred is anhydrous citric acid or a salt thereof, in particular sodium citrate, or a combination of citric acid and a citrate. It is preferred to add citrate, in particular sodium citrate to the composition to reduce foaming of the liquid during dissolution of the tablet / unit dose. A particularly preferred combination of two acid compounds is the combination of citric acid and tartaric acid.

The acidic compound in this invention is most preferably anhydrous. Further the acidic compound is preferably free of Ca and Mg ions (this means no components are included comprising Ca or Mg as counter-ions).

According to the invention the acidic component(s) of the effervescent system is/are preferably present in a total amount ranging from 10 wt.-% to 90 wt.-%, preferably in a total amount ranging from 25 wt.-% to 85 wt.-%, more preferably in a total amount ranging from 30 wt.-% to 80 wt.-%, even more preferred 40 to 70 wt.-% based on the total weight of the effervescent system.

The basic / alkaline component is preferably selected from carbonates, bicarbonates, percarbonates, sesquicarbonates, and derivatives thereof including for example sodium and potassium forms, and derivatives thereof. Preferred basic components are carbonates and/or bicarbonates, particularly preferred are sodium or potassium carbonate / bicarbonate, even more preferred are anhydrous sodium or potassium bicarbonate. These compounds can be included either separately, or in combination. According to the invention it might be preferred that the effervescent system includes at least one carbonate and at least one bicarbonate, particularly preferred at least soda or potassium carbonate and sodium or potassium bicarbonate.

The basic component(s) is/are essentially free of calcium of magnesium salts or ions, wherein "essentially free" means that the basic component comprises less than 1 % of such ions, preferably less than 0.5%, more preferred less than 0.2%, less than 0.1% or less than 0.05%. Most preferred the basic component is free of such ions.

The basic component in this invention is most preferably anhydrous.

Most preferably, the effervescent system according to the invention comprises at least citric acid and sodium bicarbonate; or at least citric acid, sodium citrate and sodium bicarbonate; or at least citric acid, sodium citrate, sodium and/or potassium carbonate and sodium bicarbonate; or consists of said components.

According to the invention the basic component of the effervescent system is preferably present in a total amount ranging 10 wt.-% to 90 wt.-%, preferably in a total amount ranging from 25 wt.-% to 85 wt.-%, more preferably in a total amount ranging from 30 wt.-% to 80 wt.-%, even more preferred 40 to 70 wt.-% based on the total weight of the effervescent system.

In a preferred embodiment the effervescent system consists of the acidic and the basic components.

The ratio of the acidic to the basic component in the effervescent system preferably ranges from 1:6 to 10:1, more preferably from 1:6 to 6:1, even more preferably from 1:2 to 6:1, most preferably from 1:1 to 4:1 (wt/wt). In a particularly preferred embodiment the acidic component is provided in excess, e.g. in a ratio of at least 1.2:1, more preferred at least 1.5:1, even more preferred at least 1.8:1, at least 2:1, or even at least 2.2:1 to the basic compound of the effervescent system.

The molar ratio of acid groups to carbonate-groups (CO₃²⁻) or bicarbonate-groups (HCO₃⁻) in the effervescent system ranges from 8:1 to 1:1, more preferably from 7:1 to 1.25:1, more preferably from 6:1 to 1.5:1, most preferably from 5:1 to 2:1. Preferably the acid groups are carboxylic acid groups.

The molar ratio of tricarboxylic acid to carbonate-groups (CO₃²⁻) or bicarbonate-groups (HCO₃⁻) in the effervescent system ranges from 2.7:1 to 0.3:1, more preferably from 2.3:1 to 0.4:1, more preferably from 2:1 to 0.5:1, most preferably from 1.7:1 to 0.7:1.

Preferably, the effervescent system according to the invention comprises a water content ranging from 0 to 5, preferably from 0 to 4, more preferably from 0 to 3, even more preferably from 0 to 2, still even more preferably from 0 to 1, still even more preferably from 0 to 0.5, and most preferably from 0 to 0.25 wt.-% of the total composition. The water content is defined by the amount of water which evaporates when the composition or components are heated to 105 °C.

### Nonionic surfactant

According to the present invention the cosmetic or body / hair care composition comprises at least one nonionic surfactant. It has been found that the presence of a nonionic surfactant, particularly at least one of the alkyl(poly)glucoside surfactants described herein just below, provides not only a suitable gentle and pleasant mild cosmetic composition, but also results in a fast and effective water absorption of the tablet / unit dose and furthermore limits the foam development, thus preventing that the liquid solution during preparation foams over the container. Therefore, it can be avoided that the user has to clean the container outer surface.

One of the most preferred nonionic surfactants are alkylglucosides, which are glucose ethers with a fatty alcohol. Particularly preferred alkylglucosides have a glucose etherified with a C₆₋₂₀ fatty alcohol, e.g. octylglucoside (caprylglucoside), decylglucoside (capringlucoside), dodecylglucoside (laurylglucoside), tetradecylglucoside (myristylglucoside), hexadecylglucoside, (cetylglucoside) or octadecylglucoside (stearylglucoside), without being limited to the mentioned .Further, alkyl polyglucosides (APG) may be produced by a reaction of a fatty alcohol with several glucose units. Preferred APGs can have averagely two to averagely four glucose units and has a hydrocarbon-chain that varies from 4 up to 26 carbon moieties, including 4, 6, 8, 10, 12, 14, 16, 18, 20, 22 C.

Another type of highly preferred nonionic surfactants are glucamide surfactants. Glucamides are the reaction product of glucose amine with a fatty acid. Particularly preferred alkylglucamides have an alkyl chain of C₆₋₂₀ hydrocarbon groups. Further, it is preferred that the glucamides have a C₁₋₃ substituent at the amid nitrogen, preferably methyl or ethyl, most preferred methyl, thus represent an N- C₁₋₃ alkyl glucamide. e.g. octoyl methyl glucamide (caproyl methyl glucamide), decoyl methyl glucamide (caprinoyl methyl glucamide), dodecoyl methyl glucamide (lauroyl methyl glucamide), tetradecoyl methyl glucamide (myristoyl methyl glucamide), hexadecoyl methyl glucamide, (cetoyl methyl glucamide) or octadecoyl methyl glucamide (stearoyl methyl glucamide), without being limited to the mentioned e.g. the glucamides offered under the tradename GlucoTain by Clariant, or Cocoyl Methyl Glucamide (INCI) or mixtures thereof.

Said alkylglucosides and / or APGs (reference to both types is herein defined as alkyl(poly)glucosides) and / or glucamides can be the only type of nonionic surfactants in the composition. If so, they can be present in an amount ranging from a total amount of 1 to 18 wt.%, preferably in an amount of 1,5 to 15 wt.%, more preferred in an amount of 2 to 13 wt.%, even more preferred in an amount of 2,5 to 12 wt.%, based on the total weight of the tablet or unit dose composition.

Besides the alkyl(poly)glucosides and/or glucamides the compositions can include further nonionic surfactants, in particular those also providing a mild and pleasant feeling to body or hair.

Further nonionic surfactants can be e.g. alkoxylated fatty alcohols, alkoxylated fatty acids or combinations thereof. The relative hydrophilic alkoxy-chain comprises or essentially consists of ethylene oxide, propylene oxide or butylene oxide or combinations thereof. Preferably said alkoxy-chain comprises or essentially consists of ethylene oxide or propylene oxide units, in particular ethylene oxide units. The length of the alkoxy chain can vary between averagely 15 and averagely 200 ethylene oxide-, propylene oxide- or butylene oxide-groups or combinations thereof, including 15, 16, 18, 20, 25, 30, 40, 50, 80, 100 and the ranges between the particularly mentioned. It is preferred that the chain comprises mainly or consists of ethylene oxide (EO). Ethoxylated fatty alcohols are particularly preferred.

The relative hydrophobic hydrocarbon-chain of the fatty alcohol or fatty acid can be saturated, mono-unsaturated or poly-unsaturated and linear or branched. The length of this hydrocarbon-chain varies between 4 and 30 carbon molecules, including 6, 8, 10, 11, 12, 14, 16, 18, 20, 22, 24, 26 or 28.

Preferred surfactants of this type may be represented by the formula R¹O-(CH₂CH₂O)ₓ-(CH(CH₃)CH₂O)_{y}-H, wherein R¹ is a linear or branched, saturated or unsaturated hydrocarbon residue having 4 to 30 C atoms, preferably 6 to 26, even more preferred 10 to 24 C atoms, x preferably is an integer of from 15 to 200 (including 16, 18, 20, 25, 30, 40, 50, 80, 100), preferably from 20 or from 25 to 100, y preferably is an integer of from 0 to at most 100 and x+y is below 200. R¹ preferably is a linear, saturated hydrocarbon residue.

Besides the type of ethoxylated fatty alcohols mentioned above or instead of them the composition also can comprise fatty alcohol ethoxylates having a short EO chain, thus, in the above formula x is an integer of from 3 to 12, preferably from 3 to 10, more preferred 4 to 8, including 5, 6 or 7 EO units, and y is 0, wherein the hydrocarbon residue is as defined before.

Particularly preferred are alcohol ethoxylates that can be prepared by ethoxylation of a fatty chain alcohol. The preferred alcohol ethoxylates have a hydrocarbon-chain that ranges from 4 up to 26 carbon molecules, including 4, 6, 8, 10, 12, 14, 16, 18, 20, 22 and between averagely 3 and 12, including 4, 5, 6, 7, 8, 9, 10 or 11 and/or between averagely 15 and averagely 200 ethylene oxide-groups, including averagely 16, 18, 20, 25, 30, 40, 50, 80, 100 ethylene oxide-groups. Ethoxylated fatty alcohols having at least 20 EO units are preferred, such having at least 25 EO units are particularly preferred. One example of such a preferred surfactant is C16-18 fatty alcohol 25 EO, e.g. commercially available as Lutensol AT25 from BASF.

Also preferred are fatty acid ethoxylates that can be prepared by a reaction of fatty acid with ethylene oxide or a polyglycol with the general formula RCOO⁻(CH₂CH₂O)ₙH. When a polyglycol is used a mixture of mono- and di-esters (RCOO⁻(CH₂CH₂O)ₙ-OCOR) is produced. The preferred fatty acid has a hydrocarbon-chain R that varies from 4 up to 26 carbon molecules, including 4, 6, 8, 10, 12, 14, 16, 18, 20, 22. The number of ethylene oxide-groups in the fatty acid ethoxylate is between averagely 15 and averagely 200 ethylene oxide-groups, including averagely 16, 18, 20, 25, 30, 40, 50, 80, 100 ethylene oxide-groups.

Further suitable nonionic surfactants are esters of any fatty acid, preferably a fatty acid comprising a hydrocarbon chain of from 6 to 20 carbon moieties with an alcohol. Said alcohol can be a mono alcohol, a diol or triol, preferably having 1 to 6 C atoms. Suitable fatty acid esters are e.g. glyceryl esters of fatty acids as defined before.

In the tablet or unit dose composition nonionic surfactant(s) described above preferably is/are present in a total amount of 1 to 25 wt.%, preferably in an amount of 1.5 to 20 wt.%, more preferred in an amount of 2 to 18 wt.%, even more preferred in an amount of 2.5 to 15 wt.%, based on the total weight amount of the tablet or unit dose composition.

### Anionic surfactant

Besides the mentioned nonionic surfactant(s) as further surfactant(s) the composition comprises at least one anionic surfactant.

Preferred anionic surfactants comprise or consist of a hydrophobic chain and an anionic hydrophilic group. The hydrophilic group can e.g. be a carboxylate, CₙH₂ₙ₊₁COO⁻ X, a sulphate, CₙH₂ₙ₊₁OSO³⁻ X, a sulphonate, CₙH₂ₙ₊₁ SO³⁻ X, a phosphate, CₙH₂ₙ₊₁OPO(OH)O⁻X, an anionic amino acid, a (sulpho)succinate, a (sulfo)acetate, an isethionate, a taurate or any other anionic hydrophilic group.

Particularly preferred types of anionic surfactants are sulfosuccinates (as mono- or di-esters or a mixture thereof) and sulfoacetates, as well as isethionates, due to their mild cleaning conditions. Examples of suitable anionic surfactants are C₆₋₂₀ alkyl sulfosuccinate, C₆₋₂₀ alkyl sulfoacetate or C₆₋₂₀ alkyl isethionate, in particular as sodium, potassium or ammonium salts. It is preferred that at least one type of these preferred anionic surfactants is present in the composition of the invention, and also a combination of two or more of the mentioned can be present in the composition.

It is particularly preferred to add at least a C₆₋₂₀ sulfosuccinate, preferably a C₁₀₋₁₆ sulfosuccinate, either as a sodium, potassium or ammonium salt, more preferred sodium lauryl sulfosuccinate, e.g. obtainable under the tradename KLK Tensomild H026 by . If suitable, the sulfosuccinate surfactant can also be ethoxylated, e.g. by 3 to 12 EO units.

Further, the addition of a sulfoacetate is preferred, either as a sole anionic surfactant, however, preferably in addition to a sulfosuccinate. If suitable, the sulfoacetate surfactant can also be ethoxylated, e.g. by 3 to 12 EO units. Examples of suitable sulfoacetates are sodium lauryl sulfoacetate (e.g. Stepan Lathanol LAL available by Stepan) or disodium laureth sulfoacetate, or a mixture of those two. Such a mixture is available by Stepan under the tradename Stepan Mild LSB.

The anionic surfactants of the type sulfosuccinate, sulfoacetate and isethionate mentioned above can be present in the composition of the invention in a total amount of from 3 to 40 wt.%, preferably in an amount of from 4 to 35 wt.%, more preferred in an amount of from 5 to 32 wt.%, even more preferred from 6 to 30 wt.%. This definition should be considered as defining that each of the mentioned anionic surfactants can be individually present in the composition in the defined range, however, the total amount of these types of surfactants is also in said range.

Examples of further optional anionic surfactants are sulfonates or sulfates, in particular C₁₀₋₂₂, preferably C₁₂₋₁₆ alkanesulfonates, C₁₀₋₂₂, preferably C₁₂₋₁₆ fatty alcohol sulfates or similar suitable anionic surfactants. Common types of sulphonate surfactants are alkyl- or alkyl aryl sulphonate (e.g. sodium C₁₄₋₁₆ alkyl sulfonate, alkyl benzene sulphonate, naphthalene sulphonate, alkyl naphthalene sulphonate), paraffin sulphonates, linear alkyl benzene sulphonate (LABS), alpha-olefin sulphonates. The properties of sulphonate or sulfate surfactants can optionally be modified by introducing ethylene oxide units in the chain. Further anionic surfactants which can be added to the composition may be alkane phosphates.

### Tablet or unit dose composition

The tablet or unit dose composition of the present invention is intended to provide a ready-to-use liquid cosmetic or body / hair care composition, in particular a cosmetic or body / hair care composition for keratinic material like skin and hair, by just adding one tablet or unit dose to a predetermined amount of water. The tablet or unit dose is therefore provided in a defined weight amount, calculated to be dissolved in a defined volume of water. To allow a pleasant handling of the ready-to-use liquid composition, the weight amount of the tablet preferably is calculated for a water volume in the range of 100 ml to 1 I, e.g. 100 ml, 150 ml, 200 ml, 250 ml, 300 ml, 400 ml, 500 ml, 600 ml, 700 ml, 750 ml, 800 ml or 1 I. The tablet or unit dose for providing the liquid composition in this volume preferably is provided in a weight amount of from 3 to 20 g, calculated such that preferably an amount of 4 to 10 g, more preferred 5 to 8 g is added to 300 ml of water. However, the amounts mentioned herein should be considered as preferred examples, not as limiting the invention to said specific amounts.

The liquid cosmetic or body / hair care composition obtained by adding one of the tablet / unit dose to water should not comprise any solid remainder(s), preferably no greasy/oily film on the surface and should be pleasantly to prepare and to use. Therefore, it is particularly preferred that the composition comprises only ingredients fully dissolvable in water in the amounts as calculated for the tablet / unit dose ingredients in the water volume as specified. Dissolution of the tablet and remaining all the ingredients of the composition dissolved during storage is noticeably increased by the use of a combination of the above-mentioned anionic and nonionic surfactants and optionally a chelating agent. These ingredients further result in a liquid mild, gentle cosmetic or body / hair care composition providing a pleasant feeling to skin and hair.

With "fully dissolvable" is meant that the ingredient(s) dissolve in the water volume as specified for the tablet / unit dose at 30°C within 10 to 15 min without any remainder due to the bubbling of the effervescent system and optional slewing (not shaking) and wherein all the ingredients remain dissolved during storage for at least 12 weeks.

The composition as provided herewith allows the preparation of a liquid or jelly, in particular aqueous cosmetic or body / hair care composition. According to the present application the liquid or jelly composition has a viscosity of from 0.5 to 10⁵ Pa s, preferably from 1 to 10⁴ Pa s, wherein "liquid" is more in the range of from 0.5 to 1000 Pa s, and "jelly" is more in the range above 1000 Pa s. In any case the liquid or jelly composition is pourable, pumpable or can be squeezed through an opening of a squeezable bottle like e.g. a shampoo or shower gel flask. The liquid or jelly composition can also be provided by pumping it, e.g. by a pump spending bottle or a pump sprayer.

It is particularly preferred that the tablet or unit dose composition is essentially free of Ca and Mg ions, thus, doesn't comprise any component including Ca or Mg, wherein "essentially free" means that the composition comprises less than 1% of such ions, preferably less than 0.5%, more preferred less than 0.2%, less than 0.1% or less than 0.05%. Most preferred the water softener composition is free of such ions.

The tablet or unit dose composition of the present invention when solved in water preferably provides a slightly acidic to neutral pH (pH 5 to pH 7, preferably pH 5,5 to pH 6,8) due to the components of the composition. This pH is obtained by applying a unit amount of a 6-9 g (e.g. 8 g) tablet into 300 ml of water (20°C) after full disintegration.

The tablet or unit dose composition according to the invention comprises a water content ranging from 0 to 5, preferably from 0 to 4, more preferably from 0 to 3, even more preferably from 0 to 2, still even more preferably from 0 to 1, still even more preferably from 0 to 0.5, and most preferably from 0 to 0.25 wt.-% of the total composition. The water content is defined by the amount of water which evaporates when the composition or components are heated to 105 °C.

### Further ingredients of cosmetic or body / hair care compositions

Preferably the cosmetic or body / hair care composition according to the invention comprises besides the effervescent system, the nonionic surfactant(s) and the anionic surfactant(s) as cited above at least one or more further component(s) selected from: chelating agent(s), further surfactants, pH adjusting ingredients, colorants, dyes, preservatives, perfumes, and optionally conditioning agents, water soluble fillers, water soluble tablet binders and disintegrants. More preferably the composition comprises at least one component selected from: chelating agents, further surfactants, pH adjusting agents and preservatives; most preferably the composition comprises at least a chelating agent and/or at least one preservative.

Said one or more additional component(s) amount(s) to a total amount of 2 to 50 wt.-% based on the total weight of the tablet or unit dose composition, preferably 5 to 45 wt.-%, more preferred 7 to 40 wt.-%, even more preferred 8 to 35 wt.-% of the total weight of the composition.

### Chelating agent

The composition of the present invention preferably contains a chelating agent. The chelating agent(s) have the double-function of chelating ions in the solution to prevent any precipitation in the liquid composition, and further to support the cleaning result of the composition. Chelating agents can include any of those known in the art, however, according to the invention are different from citric acid or citrate. This means that - if a chelating agent is present in the composition - besides the citric acid or citrate of the effervescent system a further chelating agent is present in the composition.

Preferred chelating agents are biodegradable chelating agents like amino acid-based chelating agents, in particular methyl glycine di-acetic acid (MGDA) and di-carboxymethyl glutamic acid (GLDA), preferably as alkali metal, or alkaline earth, ammonium or substitutes ammonium salts thereof, in particular as sodium salts. Another preferred biodegradable chelating agent for use herein is ethylene diamine N,N'- disuccinic acid, or alkali metal, or alkaline earth, ammonium or substitutes ammonium salts thereof or mixtures thereof. Further carboxylate chelating agents include tartaric acid, oxalic acid, salicylic acid, aspartic acid, glutamic acid, glycine, malonic acid, lactic acid or mixtures thereof.

Ethylenediamine N,N'- disuccinic acids, especially the (S,S) isomer have been extensively described in US patent 4, 704, 233. Ethylenediamine N,N'- disuccinic acids is, for instance, commercially available under the tradename ssEDDS^{®} from Palmer Research Laboratories. Further amino carboxylates that may be used herein include ethylene diamine terra acetates, diethylene triamine pentaacetates, diethylene triamine pentaacetate (DTPA),N- hydroxyethylethylenediamine triacetates, nitrilotri-acetates, ethylenediamine tetrapropionates, triethylenetetraaminehexa-acetates, ethanol-diglycines, propylene diamine tetracetic acid (PDTA), both in their acid form, or in their alkali metal, ammonium, and substituted ammonium salt forms.

Suitable phosphonate chelating agents are diethylene triamine penta methylene phosphonate (DTPMP) and ethane 1-hydroxy diphosphonate (HEDP). Such phosphonate chelating agents are commercially available from Monsanto under the trade name DEQUEST^{®}-

Further chelating agents are ethylenediamine-N,N'-tetraacetic acid (EDTA) or ethylenediamine-N,N'-disuccinic acid (EDDS) or the alkali metal, alkaline earth metal, ammonium, or substituted ammonium salts thereof, or mixtures thereof. Preferred EDTA or EDDS compounds are the free acid form and the sodium salt or complex thereof.

Still other chelating agents are iminodiacetic acid derivatives such as 2-hydroxyethyl diacetic acid or glyceryl imino diacetic acid, described in EP 0317542 A2 and EP 0399133 A1.

Typically, the tablet or unit dose compositions according to the present invention comprise a total amount of from 0,1 up to about 15 wt.%, preferably from 0,3 to 10 wt.%, more preferably from 0,5 to 8 wt.%, even more preferred from 0,8 to 5 wt.% or 1 to 3 wt.% of a chelating agent, or a mixture of chelating agents (different from citric acid or citrate) based on the total weight amount of the tablet or unit dose composition Preferably the compositions comprise only biodegradable chelating agents, most preferred selected from MGDA and GLDA, or a combination of those.

### Additional Surfactants

Besides the nonionic and the anionic surfactants mentioned above as necessary ingredient the body / hair care or cosmetic composition may comprise additionally a total amount of 0.5 to 30 wt.-%, preferably 1 to 20 wt.-%, 2 to 15 wt.-%, 2.5 to 12 wt.-% or 3 to 10 wt.-% of any further surfactant(s). The mentioned ranges refer to the total amount of the added additional surfactants, thus, if a mixture of surfactants is comprised in the composition the sum of the added amounts shall be inside the mentioned ranges and refers to the total amount of the tablet or unit dose composition.

Suitable surfactants for additional use in the composition herein may be further nonionic surfactants (differing from them cited above), gemini surfactants, amphoteric surfactants, anionic surfactants, zwitterionic surfactants, cationic surfactants or a combination thereof, wherein anionic and non-ionic surfactants are particularly preferred.

One type of particularly preferred additional surfactants are further nonionic surfactants Suitable further nonionic surfactants are e.g. alkyl amine oxides, in particular amine oxides having one alkylene chain with C₆₋₂₅, including 7, 8, 9, 10, 11, 12, 13, 14, 15, 16, 17, 18, 19, 20, 21, 22, 23 or 24 carbon atoms, and two short alkyl chains including 1, 2 or 3 carbon atoms. A particularly preferred amine oxide is N-dodecyl-N,N-dimethyl amine oxide.

Further suitable nonionic surfactants are alkoxylated fatty alcohols or alkoxylated fatty acids, wherein the alkoxy-chain comprises or essentially consists of less than 15 ethylene oxide, propylene oxide or butylene oxide units or combinations thereof. Preferably said alkoxy-chain comprises or essentially consists of ethylene oxide or propylene oxide units, in particular ethylene oxide units. The length of the alkoxy chain of the additional surfactants is below 15 and preferably ranges from 2 to 14, more preferred from 3 to 10, even more preferred from 3 to 8, including 4, 5, 6, and 7. It is preferred that the chain comprises mainly or consists of ethylene oxide. Ethoxylated fatty alcohols are particularly preferred.

The hydrocarbon-chain of the fatty alcohol or fatty acid can be saturated, mono-unsaturated or poly-unsaturated and linear or branched. The length of this hydrocarbon-chain varies between 4 and 30 carbon molecules, including 6, 8, 10, 11, 12, 14, 16, 18, 20, 22, 24, 26 or 28.

Nonionic surfactants can be "end-capped" by a methyl or an ethyl group at the end of the alkoxy-chain. This affects the properties of the surfactant, e.g. it decreases the foaming behavior. It is also known that nonionic surfactants can be "end-capped" with a fatty alcohol or a fatty acid where the hydrocarbon-chain varies from 4 up to 26 carbon molecules, including 4, 6, 8, 10, 11, 12, 14, 16, 18, 20, 22. The structure of the surfactants than is "hydrocarbon chain - alkoxy chain - hydrocarbon chain".

Further preferred as additional nonionic surfactants are amine ethoxylates that can be prepared by addition of ethylene oxide to primary or secondary fatty amines. With primary amines both hydrogen atoms on the amine group react with ethylene oxide. These surfactants have a cationic character when there are a few ethylene oxide units and the pH is low. Under alkaline conditions the surfactant is water soluble when the alkyl chain is not long.

Further suitable are ethylene oxide-propylene oxide co-polymers which may be prepared with a starting material that reacts with ethylene oxide (EO) or propylene oxide (PO) or a mixture of EO and PO (resulting in block copolymers). The starting materials that can be used are (difunctional) poly(oxypropylene glycol) or (difunctional) poly(oxyethylene glycol) or glycerol (for trifunctional products) or ethylene diamine (for tetrafunctional products).

Further optional additional surfactants are cationic surfactants. Such surfactants have typically a quartery nitrogen and usually four alkyl chains independently with C₁ to C₂₀. Examples for the alkyl chains are independently methyl-, ethyl-, propyl-, butyl- as typical short chains and lauryl-, myristyl-, palmityl- or stearyl-chains as typical long chains. A typical cationic surfactant has at least one short chain, often two short chains e.g. methyl, and at least two longer chains, independently selected from lauryl-, myristyl-, palmityl- or stearyl-chains.

Also, amphoteric (zwitterionic) surfactants can be additionally added. The most representative amphoteric surfactants are betains or sulphobetains.

Preferred gemini-surfactants depend of the chosen tail, ion-group and spacer the Gemini-surfactant can have different properties. The tail can for example consists of glucoside, poly-glucoside or a hydrocarbon chain. The hydrocarbon chain can be saturated or unsaturated, branched or linear. The ion-group can be cationic (e.g. ammonium), anionic (e.g. phosphate, sulphate, carboxylate) or nonionic. The spacer can be short or long methylene groups, rigid (stilbene), polar (polyether, polyethyleenoxide), and nonpolar (aliphatic, aromatic).

### pH adjusting agents

The preferred slightly acidic pH of the liquid cosmetic or body / hair care composition may directly result from the ratio of the acidic and basic components of the effervescent system. Nonetheless, it might be further suitable to adapt the pH of the solution by adding further pH adjusting agents to the tablet or unit dose composition.

By "acidifier/acidification agents" herein it is meant any component which when released, acts such as to reduce the pH of the obtained solution containing the dissolved/dispersed composition. Suitable acidification agents for use in the composition of the present invention include inorganic and organic acids including, for example, carboxylate acids except citric acid or citrate, like e.g. succinic acids, tartaric acid, oxalic acid, or polycarboxylate acids, such as polyacrylic acid, and also acetic acid, boric acid, malonic acid, adipic acid, fumaric acid, lactic acid, glycolic acid, maleic acid, their derivatives and any mixtures of the foregoing.

Alkaline agents for raising the pH up to 7 - besides the alkaline / basic components of the effervescent system - might be selected from soap, NaOH and KOH.

The pH adjusting agents are added in an amount sufficient to reach the desired pH in the solution prepared by dissolving the tablet or unit dose composition according to the invention in water as described above.

### Perfumes

Suitable perfumes include blooming perfumes, perfume oils, and perfume raw materials comprising alcohols, ketones, aldehydes, esters, ethers, nitriles alkenes, and mixtures thereof. Examples of ester type perfumes include benzylacetate, phenoxyethylisobutyrat, p-tert.-butylcyclohexylacetat, linalylacetat, dimethylbenzyl-carbinylacetate, phenylethyl acetate, linalyl benzoate, benzylformiate, ethylmethylphenyl glycinate, allylcyclohexyl propionate, styrallyl propionats and benzylsalicylate. Examples for ether type perfumes are benzylethylether, and for example linear C₈₋₁₈ alkanals, citral, citronellal, citronellyl oxyacetaldehyde, cyclamenaldehyde, hydroxycitronellal, lilial and bourgeonal, examples for ketones are ionones, alpha -isomethylionone and methyl-cedrylketone, examples for alcohols are anethol, citronellol, eugenol, geraniol, linalool, phenylethylalkohol and terpineol, examples for carbohydrates are terpene including limonen and pinen. Mixtures of different perfumes generating distinct fragrances may be preferred. These may include naturally occurring oils such as for example from pine, citrus, jasmine, patchouli, rose, ylang ylang, sage, camomille, cloves, balm, mint, orange flower, orange peel, sandel wood, neroliol, cinnamon, lime-tree blossom, juniper berry, vetiver, olibanum, galbanum and labdanum.

If present, the perfumes may preferably be present in an amount of up to 3 wt.-%, more preferably in an amount of from 0,1 to 2,5 wt.-%, based on the total weight of the composition.

### Colorants/dyes

Suitable colorants and dyes include all kinds of colorants and dyes Particularly suitable are colorants which are water soluble or soluble in at ambient temperature liquid organic solvents.

A preferred type of colorants are polymeric colorants, sold by Milliken under the name "liquitint^{®}" in several colours.

Further suitable examples of colorants include anionic dyes like for example anionic nitroso dyes. One possible suitable dye is naphthol green (Colour Index (CI) part 1: Acid Green 1; Teil 2: 10020), which is commercially available as Basacid TM Green 970 from BASF, (Ludwigshafen, Germany) and mixtures thereof with suitable blue dyes. Further suitable colorants are for example Pigmosol TM Blue 6900 (CI 74160), Pigmosol TM Green 8730 (CI 74260), Basonyl TM Red 545 FL (CI 45170), Sandolan TM Rhodamin EB400 (CI 45100), Basacid TM Yellow 094 (CI 47005), Sicovit TM Patentblue 85 E 131 (CI 42051), Acid Blue 183 (CAS 12217-22-0, CI Acidblue 183), Pigment Blue 15 (CI 74160), Supranol TM Blue GLW (CAS 12219-32- 8, CI Acidblue 221), Nylosan TM Yellow N-7GL SGR (CAS 61814-57-1, CI Acidyellow 218) und/oder Sandolan TM Blue (CI Acid Blue 182, CAS 12219-26-0).

If present, the amount of colorants is preferably from 0,01 to below 1 wt.%, preferably below 0.5 wt.-%, more preferably below 0.1 wt.-%.

### Preservatives

Suitable preservatives for use herein include compounds with antimicrobial effect. If present suitable preservatives according to the present invention may be selected from the group including alcohols, aldehydes, antimicrobial acids, carbonic acid esters, acid amides, phenols, phenol derivatives, sorbates like sodium or potassium sorbate, diphenyls, diphenyl alkans, urea derivatives, oxygen-nitrogen-acetales, formates, benzamidines, substituted isothiazoles, and hydrated isothiazol derivatives, such as for example isothiazolinen and isothiazolidinen, phthalimid derivatives, pyridine derivatives, antimicrobial surfactants, such as for example antimicrobial quarternary surfactants, guanidines, antimicrobial amphoteric compounds, chinoline, 1,2-dibrom-2,4-dicyanobutane, iodo-2-propynyl-butyl-carbamate, iodine, iodophore and peroxide including for example phenoxyethanol, undecylene acid, salicylic acid, benzoeic acid or their sodium or potassium salts, in particular sodium benzoate, 2-benzyl-4-chlorphenol, 2,2'-methylen-bis-(6-brom-4-chlorphenol), 2,4,4'-trichlor-2'-hydroxydiphenylether, N-(3-aminopropyl)-N-dodecylpropame-1,3-diamine, N-(4-chlorphenyl)-N- (3,4-dichlor-phenyl)-urea, N,N'-(1,10-decandiyldi-1-pyridinyl-4-yliden)-bis-(1-octanamin)-dihydro-chlorid and N,N'-bis-(4-chlorphenyl)-3,12-diimino-2,4,11,13-tetraaza-tetradecandiimid-amide, as for example disclosed in H. Wailhäusser "Praxis der Sterilisation, Desinfektion, Konservierung, Keimidentifizierung, Betriebshygiene" (5th edition, Stuttgart; New York: Thieme, 1995. Particularly preferred preservatives are potassium sorbate and/or sodium benzoate, preferably in a total amount of 1 to 7 wt.%, more preferred 2 to 6 wt.% of the total weight of the tablet or unit dose composition. More preferred both said preservatives are present, preferably in a ratio of sorbate to benzoate of 1:1 to 1:2, preferably 1:1.2 to 1:1.8, more preferred 1:1.3 to 1:1.6, like e.g. 1:1.5.

### Conditioning agents / Cationic polymers

Dependent from the intended use, the composition further can comprise cationic polymers e.g. those known as conditioning agents for hair treatment.

Non-limiting examples of cationic polymers include poly(methacryloyloxyethyl trimethylammonium chloride), polyquaternium-37, quaternized cellulose derivatives, polyquaternium-4, polyquaternium-10, cationic alkyl polyglycosides, cationized honey, cationic guar derivatives, polymeric dimethyl diallyl ammonium salts and copolymers thereof with esters and amides of acrylic acid and methacrylic acid, copolymers of vinyl pyrrolidone with quaternized derivatives of dialkylaminoalkyl acrylate and methacrylate, vinyl pyrrolidone-vinyl imidazolium methochloride copolymers, quaternized polyvinyl alcohol, polyquaternium-2, polyquaternium-7, polyquaternium-17, polyquaternium-18, polyquaternium-24, polyquaternium-27, and a mixture thereof. In some instances, the one or more cationic polymers may be selected from the group consisting of polyquaternium-4, polyquaternium-10, cationic guar derivatives, and a mixture thereof.

The cationic polymers can be a monoalkyl quaternary amine, such as stearyltrimonium chloride, soyatrimonium chloride or coco-ethyldimonium ethosulfate. Other suitable cationic polymers include, but are not limited to, behentrimonium chloride, dialkyl quaternary amines, such as dicetyldimonium chloride, dicocodimethyl ammonium chloride or distearyldimethyl ammonium chloride; and polyquaternium compounds, such as Polyquaternium-6, Polyquaternium-22 or Polyquaternium-5.

For example, cationic polymers may be chosen from polyquaterium-10 (also called quaternized polyhydroxyethyl cellulose), cetrimonium chloride (also called cetyl trimethyl ammonium chloride, CTAC), behentrimonium chloride (also known as docosyl trimethyl ammonium chloride), behentrimonium methosulfate, steartrimonium chloride, stearalkonium chloride, dicetyldimonium chloride, hydroxypropyltrimonium chloride, cocotrimonium methosulfate, olealkonium chloride, steartrimonium chloride, babassuamidopropalkonium chloride, brassicamidopropyl dimethylamine, Quaternium-91, Salcare/PQ-37, Quaternium-22, Quaternium-87, Polyquaternium-4, Polyquaternium-6, Polyquaternium-11, Polyquaternium-44, Polyquaternium-67, amodimethicone, lauryl betaine, Polyacrylate-1 Crosspolymer, steardimonium hydroxypropyl hydrolyzed wheat protein, behenamidopropyl PG-dimonium chloride, lauryldimonium hydroxypropyl hydrolyzed soy protein, aminopropyl dimethicone, Quaterium-8, and dilinoleamidopropyl dimethylamine dimethicone PEG-7 phosphate.

In some instances, the cationic polymers are cationic conditioning polymers. Examples of cationic conditioning polymers that can be used include, without limitation, cationic cellulose, cationic starch, cationic guar gums, cationic proteins, and cationic polymers. The cationic polymers can have a vinyl group backbone of amino and/or quaternary ammonium monomers. Cationic amino and quaternary ammonium monomers include, without limitation, dialkylamino alkylmethacrylate, monoalkylaminoalkyl acrylate, monoalkylaminoalkyl methacrylate, trialkyl methacryoloxyalkyl ammonium salt, trialkyl acryloxyalkyl ammonium salts, diallyl quaternary ammonium salts, vinyl compounds substituted with dialkyl aminoalkyl acrylate, and vinyl quaternary ammonium monomers having cyclic cationic nitrogen containing rings such as pyridinium, imidazolium, or quaternized pyrrolidine. Other examples of cationic conditioning polymers that can be used include, without limitation, hydroxypropyltrimonium honey, cocodimonium silk amino acids, cocodimonium hydroxypropyl hydrolyzed wheat or silk protein, polyquaternium-5, polyquaternium-11, polyquaternium-2, polyquaternium-4, polyquaternium-6, polyquaternium-7, polyquaternium-14, polyquaternium-16, polyquaternium-22, polyquaternium-10, and guar hydroxypropyltrimonium chloride.

In some cases quaternized polymeric cationic polymers are particularly useful. Particularly preferred are quaternary nitrogen polymers prepared by the polymerization of a dialkyldiallylammonium salt or copolymer thereof in which the alkyl group contains 1 to about 18 carbon atoms, and more preferably where the alkyl group is methyl or ethyl. Details concerning the preparation of these polymers can be found in U.S. Pat. Nos. 3,288,770, 3,412,019 and 4,772,462. For example, cationic homopolymers and copolymers of polydiallyldimethylammonium chloride are available in aqueous compositions sold under the trademark MERQUAT by the Calgon Corporation, subsidiary of Merck and Co., Pittsburgh, Pa. The homopolymer, which is named Polyquaternium-6 is sold under the trademark MERQUAT-100, and is described as having a weight average molecular weight of approximately 100,000. A copolymer reaction product of dimethyldiallylammonium chloride with acrylamide monomers is named Polyquaternium-7 is described as having a weight average molecular weight of approximately 500,000 and is sold under the trademark MERQUAT-550. Another copolymer reaction product of dimethyldiallylammonium chloride with acrylic acids having a weight average molecular weight from about 50,000 to about 10,000,000 has the name Polyquaternium-22 and is sold under the trademark MERQUAT-280. Polyquaternium-6 is particularly preferred.

Smaller molecule cationic non-polymeric conditioning agents can also be utilized herein. Exemplary small-molecule conditioning agents can include monofunctional or difunctional quaternary ammonium compounds, such as stearyldimethylbenzylammonium chloride, dimethyldi-(hydrogenated tallow)ammonium chloride, and the like. Non-polymeric conditioning agents can also include the quaternary ammonium salts of gluconamide derivatives, such as gamma-gluconamidopropyldimethyl-2-hydroxyethyl-ammonium chloride and minkamidopropyldimethyl-2-hydroxyethylammonium chloride identified respectively by the names Quaternium 22 and Quaternium 26.

Conditioning agents - if present - preferably are contained in the tablet / unit dose composition in a total amount of 0.5 wt.% to 20 wt.%, preferably in an amount of from 1 wt.% to 15 wt.%, more preferred 2 wt.% to 12 wt.% of the total weight of the composition.

### Tablet binders/disintegrants

Materials which are known to be binders and/or disintegrants for tablets include organic materials such as starches, for example, corn, maize, rice and potato starches and starch derivatives, such as carboxymethyl starch, modified or pregelatinized starch and sodium starch glycolate; celluloses and cellulose derivatives, for example, carboxymethyl cellulose, cross-linked modified cellulose, and microcrystalline cellulose; pectin, sugars (for example, glucose, sucrose, lactose, mannitol, sorbitol, dextran, maltodextrin, mannitol,); carrageenan, alginate; shellac; xanthan gum; naturally derived waxes like bees wax and hydrogenated jojoba wax, carnauba and rice bran wax and synthetic waxes, paraffin, talcum, gelatine, lanolin, and various synthetic organic polymers, notably polyethylene glycol and derivatives, polyvinylalcohol, polyvinylacetate, polyvinyl pyrrolidone, including for example cross-linked polyvinyl pyrrolidone, poly acrylic acid, including crosslinked polyacrylic acid; copolymers of acrylic acid and copolymers of methacrylic acid or derivatives thereof; polyacrylate; and derivatives and/or mixtures thereof, all of them only as far as they are water dissolvable. If present, the composition of the present invention may also comprise more than one binder/disintegrant. According to the invention, however, it is preferred not to include any polyacrylates into the tablet / unit dose of the present invention. Further it is preferred to include less than 30 wt.%, preferably less than 20 wt.%, more preferred less than 10 wt.%, and even more preferred no starch or cellulose (derivatives) to the composition, or even no polysaccharide at all, to avoid any non-soluble residues or any precipitation during storage of the ready-prepared liquid or jelly composition.

The compound preferably used as a binder (and further as a "whetting compound") for the solid components of the composition before they are pressed together to prepare the tablet / unit dose of the present invention is glycerol, i.e. 1,2,3 propane triol.

The binder/disintegrant - if present - is preferably used in a combined amount within the range of from 0.01 to 10 wt.-%, more preferably from 0,1 to 8 wt.-%, even more preferred from 0,4 to 6 wt.%, most preferably from 0,5 to 5 wt.-% based on the total weight of the tablet or unit dose composition.

### Kit of use: tablet / unit dose and container for the liquid composition

The present invention further refers to a kit comprising at least one container for preparing the liquid cosmetic or body / hair care composition therein by adding a tablet or unit dose of the composition to a specified volume of water.

Said kit comprises at least one tablet or unit dose of the composition of the present invention and a container. The kit preferably comprises at least 2, more preferred at least 3, at least 5, at least 8 or at least 10 tablets or unit doses of the composition of the present invention and a container.

Said container preferably is adapted to the intended use, e.g. can be provided with a pull-to open cap, a push-to-open cap, a turn-to-open cap, a spray head, a dosing system etc. Further, the container provides an opening designed concerning size and shape so that the tablet or unit dose can be fed through and/or included into the container without any difficulties. Preferably the container comprises the opening at the top, which after addition of the tablet or unit dose is closed with the cap or head suitable for use.

The tablet or unit dose may be provided in any suitable package.

### Use/application of the liquid or jelly composition

The liquid or jelly composition prepared by addition of the tablet / unit dose of the present invention to water is intended for use as a cosmetic composition, in particular for body or hair treatment, e.g. cleansing, and can be used as a shampoo, hair conditioner, shower gel, bathing composition, facial cleanser, hand or body cleansing or any other suitable cosmetic composition for body or hair treatment. With body or hair treatment is meant any treatment, in particular cleansing of any part of the body surface, like the skin, nails or lips, or keratinic fibers.

### EXAMPLE

Tablets for body / hair care compositions have been prepared having the compositions as shown in table 1. Each of the tablets had a weight of 8 g and they have been used to prepare body / hair care compositions by adding any of the tablets into 300 ml of water. Properties of the liquid body / hair care compositions are determined.

**Table 1. Formulations of tablets amounts in wt.%**

| Formulation | A | B | C |
|---|---|---|---|
| Citric acid | 30-40 | 30-40 | 30-40 |
| tartaric acid | 6-15 | 6-15 | 6-15 |
| Sodium carbonate | 8-20 | 8-20 | 8-20 |
| Sodium bicarbonate | 2-12 | 2-12 | 2-12 |
| Pottash | 0-12 | 0-12 | 0-12 |
| | | | |
| alkylglucoside (1) | 2,5-15 | | 1-10* |
| glucamide (2) | | 2,5-15 | 1-10* |
| anionic surfactant (3) | 5-30 | 5-30 | 5-30 |
| nonionic fatty alcohol (4) | 0-6 | 0-6 | 0-6 |
| kationic surfactant betaine | 0-5 | 0-5 | 0-5 |
| MGDA | 0-2,5 | 0-2,5 | 0-2,5 |
| Potassium sorbate | 0-2 | 0-2 | 0-2 |
| Sodium benzoate | 0-3 | 0-3 | 0-3 |
| glycerol | 0,2-3 | 0,2-3 | 0,2-3 |
| perfumes, colorants, minors | Ad 100 | Ad 100 | Ad 100 |
| Foaming during preparation | low | low | low |
| pH of liquid composition | 5,5-7 | 5,5-7 | 5,5-7 |
| Oily film | no | no | No |
| Solid remainders | no | no | No |

## Claims

1. A cosmetic or body / hair care composition in form of a tablet or unit dose for preparing a ready-to-use liquid cosmetic or body / hair care composition, said tablet or unit dose comprising:
(i) an effervescent system, and
(ii) an anionic surfactant, and
(iii) a nonionic surfactant.

2. The tablet or unit dose according to claim 1, wherein the effervescent system is present in the tablet / unit dose in an amount ranging from 25 wt.-% to 95 wt.-%, preferably in an amount ranging from 30 to 90 wt.-% and comprises at least one acidic component selected from tricarboxylic acids, dicarboxylic acids or inorganic acids and at least one basic component.

3. The tablet or unit dose according to claim 2, wherein the effervescent system comprises at least citric acid and sodium bicarbonate, preferably at least citric acid, sodium bicarbonate and sodium and/or potassium carbonate.

4. The tablet or unit dose according to any of the preceding claims, wherein at least one of the anionic surfactant(s) (ii) is/are selected from sulfosuccinates, sulfoacetates and isethionates.

5. The tablet or unit dose according to any of the preceding claims, wherein at least one of the nonionic surfactant(s) (iii) is/are selected from alkylglucosides, alkylpolyglucosides or glucamides.

6. The tablet or unit dose according to any of the preceding claims, wherein
(a) the anionic surfactant(s) (ii) is/are present in a total amount of from 3 to 40 wt.%, preferably in an amount of from 4 to 35 wt.%, more preferred in an amount of from 5 to 32 wt.%, even more preferred from 6 to 30 wt.%; and / or
(b) the nonionic surfactant(s) (iii) is/are present in a total amount of 1 to 25 wt.%, preferably in an amount of 1.5 to 20 wt.%, more preferred in an amount of 2 to 18 wt.%, even more preferred in an amount of 2.5 to 15 wt.% of the total weight of the tablet or unit dose.

7. The tablet or unit dose according to any of the preceding claims, further comprising a chelating agent, preferably in a total amount of from 0,1 to 15 wt.%, preferably from 0,3 to 10 wt.%, more preferred from 0,5 to 8 wt.%, even more preferred from 0,8 to 5 wt.% or 1 to 3 wt.%.

8. The tablet or unit dose according to claim 7, wherein the chelating agent is selected from any biodegradable chelating agent, preferably from MGDA, GLDA or ethylene diamine N, N'-disuccinic acid, in particular their alkali metal, alkaline earth, or ammonium salts, preferably their sodium salts; or from phosphonates.

9. The tablet or unit dose according to any of the preceding claims, wherein the tablet / unit dose
(a) comprises a water soluble tablet binder, preferably said binder is glycerol; and / or
(b) is free of any polyacrylate; and / or
(c) comprises less than 30 wt.% starch or cellulose (derivatives)
(d) comprises at least one preservative.

10. The tablet or unit dose according to any of the preceding claims, wherein the tablet or unit dose comprises at least one further ingredient selected from further surfactants, pH adjusting ingredients, colorants, dyes, perfumes, water soluble fillers conditioning agents and disintegrants.

11. The tablet or unit dose according to any of the preceding claims, wherein the tablet or unit dose comprises at least one further surfactant(s), selected from further anionic surfactant(s) differing from anionic surfactants (ii), in particular differing from sulfosuccinates, sulfoacetates and isethionates, further nonionic surfactant differing from the nonionic surfactant(s) (iii), in particular differing from alkyl (poly)gucosides or glucamides, cationic or amphoteric surfactant(s), wherein further nonionic and/or anionic surfactants are preferred.

12. Use of a tablet or unit dose as defined in any of the preceding claims for preparing a liquid cosmetic or body / hair care composition, preferably by adding at least one table or unit dose to a specified volume of water.

13. A liquid or jelly cosmetic or body / hair care composition prepared by addition of a tablet or unit dose of a cosmetic or body / hair care composition as defined in any of the preceding claims into a specified volume of water.

14. A kit for preparing a liquid cosmetic or body / hair care composition comprising at least one tablet or unit dose as defined in any of claims 1 to 12 and a container having an opening shaped and sized to feed said tablet or unit dose through said opening and a cap or head for closing said opening of the container.

15. Kit according to claim 14, wherein the cap or head of the container comprises a push-to-open, a pull-to-open or a turn-to-open cap or a spraying head or a dosing system.
